# EUROPEAN PATENT SPECIFICATION

(11) **EP 1 773 399 B1**
(45) Date of publication and mention of the grant of the patent: **27.02.2008**
(21) Application number: 05760579.2
(22) Date of filing: 23.06.2005
(51) Int. Cl.: A61K 47/48, A61K 47/36

(54) **HYDROGELS OF HYALURONIC ACID AND ALPHA, BETA-POLYASPARTYLHYDRAZIDE AND THEIR BIOMEDICAL AND PHARMACEUTICAL USES**
HYALURONSÄURE UND ALPHA-, BETA-POLYASPARTYLHYDRAZIDHYDROGELE UND DEREN BIOMEDIZINISCHEN UND PHARMAZEUTISCHEN ANWENDUNGEN
HYDROGELS D'ACIDE HYALURONIQUE ET DE POLYASPARTYL-HYDRAZIDE ALPHA, BETA ET LEURS UTILISATIONS PHARMACEUTIQUES ET BIOMEDICALES

(30) Priority: 28.06.2004 IT RM20040318
(43) Date of publication of application: 18.04.2007
(73) Proprietor: Universita' degli Studi di Palermo, 90133 Palermo (IT)
(72) Inventor: GIAMMONA, Gaetano, I-90133 Palermo (IT); PITARRESI, Giovanna, I-90133 Palermo (IT); PALUMBO, Fabio, Salvatore, I-90133 Palermo (IT)
(74) Representative: Banchetti, Marina
(86) International application number: PCT/IT2005/000364
(87) International publication number: WO 2006/001046

(56) References cited:
- WO-A-02/41877
- US-A- 5 616 568
- US-A- 5 874 417
- PRESTWICH G D ET AL: "Controlled chemical modification of hyaluronic acid: synthesis, applications, and biodegradation of hydrazide derivatives" JOURNAL OF CONTROLLED RELEASE, ELSEVIER SCIENCE PUBLISHERS B.V. AMSTERDAM, NL, vol. 53, no. 1-3, 30 April 1998 (1998-04-30), pages 93-103, XP004121260 ISSN: 0168-3659
- GIAMMONA G ET AL: "A NEW WATER-SOLUBLE SYNTHETIC POLYMER, , -POLYASPARTHYDRAZIDE, AS POTENTIAL PLASMA EXPANDER AND DRUG CARRIER" JOURNAL OF CONTROLLED RELEASE, ELSEVIER SCIENCE PUBLISHERS B.V. AMSTERDAM, NL, vol. 29, no. 1/2, 1 February 1994 (1994-02-01), pages 63-72, XP000433652 ISSN: 0168-3659 cited in the application

## Description

The present invention relates to new hydrogels of hyaluronic acid and alpha,beta-polyaspartylhydrazide and their applications in the biomedical and pharmaceutical field. In particular, this invention concerns products and compositions based on chemical crosslinking of hyaluronic acid with a multifunctional biocompatible polymer with a protein-like structure bearing hydrazido pendent groups along the polymeric chain. Following this crosslinking it is possible to obtain materials characterized by a strong resistance to chemical and enzymatic degradation, unlike the starting hyaluronic acid, that can be utilized to prepare systems for biomedical and pharmaceutical applications.

As it is known, hydrogels consist of natural polymers or their derivatives, synthetic polymers or combinations of natural and synthetic polymers, whose molecules, interacting by electrostatic forces or chemical linkages, form hydrophilic crosslinked polymers, able to take up water in an amount ranging from 10-20% to several hundreds of times their dry weight. Due to their hydrophilic properties, together with their potential biocompatibility, hydrogels attract an increasing interest in the pharmaceutical and biomedical field.

In particular, hydrogels are ideal candidates in the preparation of tissue engineering matrices, with the aim to heal or reconstruct *ex novo* damaged, diseased or deteriorated human tissues or organs. Tissue engineering is actually a new science dealing with the development of technologies useful to obtain a regeneration of human damaged tissues or their complete reproduction. To allow growing and differentiation of repairing tissue cells (e.g. fibroblasts for cutaneous tissue, chondrocites for cartilaginous tissue, osteoblasts for bone tissue, etc.) and subsequent deposition of extracellular matrix (ECM, that is the principal component of all tissues), three-dimensional structures are needed, specifically porous systems, wherein cells can find an environment similar as much as possible to the natural one and they can adhere, multiply and deposit new ECM. These three-dimensional structures are usually referred to as "scaffolds", and it is already known that hydrogels are particularly suitable to constitute similar matrices for tissue engineering, considering their several advantages, for an example, compared to such hydrophobic structures. In particular, these advantages include the ability of hydrogels to allow a good fluxing of nutrients to cells and refluxing of products outside the cells, their usual biocompatibility and progressive bio-reabsorption and their ability to easily incorporate peptide ligands for cellular adhesion by covalent or physical linkages, in order to stimulate adhesion, proliferation and growing of the cells inside the hydrogel matrix. The latter advantage makes hydrogels different, for example, from hydrophobic polymers applied for the same purpose, such as PLGA (polylactic-co-glycolic acid). On the other hand, hydrogels can suffer the disadvantage of a low mechanical resistance that can reduce handling, or they may be also difficult sterilize.

The tissue engineering applications include the opportunity to use biodegradable sponges or biodegradable films for articular cartilage regeneration, or to protect and support healing of wounds caused by trauma (i.e. bums) or diseases (diabetes, AIDS). In this case the application on wounds can support a faster regenerative activity of fibroblasts that, adhering on the scaffold, will synthesize more rapidly new ECM and heal the wound. As an alternative, the scaffold can be at first utilized *in vitro* to create a real artificial derma that can be subsequently used to cover the wound and to perform its function. Similar skin substitutes perform a temporary cover able to reduce the exudates loss from the wound and the infection risk. The scaffold, when opportunely loaded with a drug, can also perform a drug delivery function, supporting as an example the prolonged release of antibiotics or growth factors, depending on type of wound. As an example, several products obtained from collagen based scaffolds are already marketed as skin substitutes (in particular a cellular bilayer obtained by growing of fibroblasts and keratinocites on collagen scaffolds, marketed with the trade mark Apligraft™).

Another biomedical application of hydrogels, having great interest for its potentialities, is the use in prevention of post-surgical adhesion. As it is known, the post-surgical adhesions consist in the formation of fibrous sutures between two opposite tissue surfaces, resulting from the trauma that tissues suffer during surgical activities. These post-surgical adhesions are a prominent problem not only in cardiovascular surgery but also in gastrointestinal and gynaecological surgery, where they can produce intestinal obstruction, infertility and pelvic pain. The most used method to prevent this bothersome problem is the positioning of biocompatible materials as physical barriers between tissues in touch, suitable to favour their complete separation and able to remain in place during the whole critical post-surgical period.

Among the barriers already marketed for this use, the barrier realized using expanded polytetrafluoroethylene (Preclude^{™}, W. L. Gore, Flagstaff, AZ), proposed as pericardial substitute, has a good clinical efficacy but is not completely bioreabsorbable, and therefore a second surgical operation is necessary for its removal. Regenerated cellulose-based barriers (Intercede^{™}, Johnson & Johnson Medical Inc., Arlington, TX), are also used but they have shown a good efficiency only if used avoiding blood contact. However at present the most widely marketed antiadhesion barrier is Seprafilm® (Genzyme, Cambridge, MA); it is a material based on hyaluronic acid modified with carboxymethylcellulose. Despite its favourable characteristics, this material shows a reduced ease of handling, it is brittle and not very elastic, and it is characterized by quite fast reabsorption times in spite of the chemical modification produced on hyaluronic acid.

As known, an excellent candidate for this and other biomedical and pharmaceutical applications reported below is hyaluronic acid, a linear polysaccharide with a high molecular weight composed of alternating units of D-glucuronic acid (GIcUA) and N-acetyl-D-glucosamine (GIcNAc), whose chemical structure can be represented by the following formula: 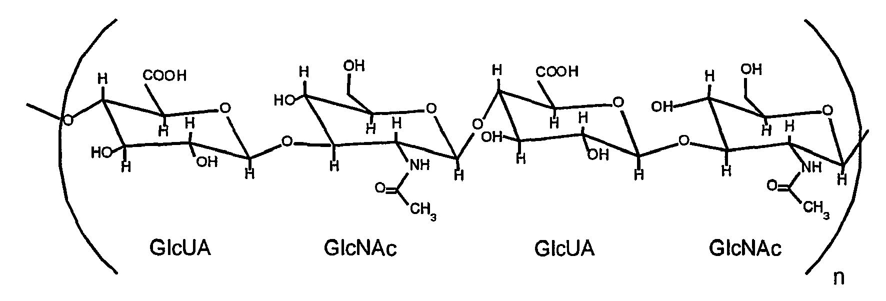 where two subsequent disaccharide units are showed, and where the number n of couples of repeating units is such that the polysaccharide molecular weight is between 50.000 and millions of dalton.

Hyaluronic acid (HA) is extensively diffused in animal tissues, being a fundamental component of the extracellular matrix, where it acts regulating the cellular proliferation and differentiation. It takes part in several important biological processes, such as cellular mobility and tissue healing; it regulates the inflammatory response and acts as a "scavenger" of free radicals. It has been demonstrated that HA is involved in tumour growth by interacting with specific receptors placed on the cell surface: this may explain the recent interest that this polymer has caused for a possible application as a soluble carrier in the production of new macromolecular prodrugs with an antitumoral activity.

Generally, hyaluronic acid is applied in viscosupplementation - both as pharmaceutical agent and as surgical aid - in the ophthalmic field, and it is widely tested in viscosupplementation to relieve articular pains caused by osteoarthritis of different nature, as a lubricating agent administered by intra-articular injections, it is applied as a "drug delivery system", i.e. as a carrier for the prolonged or controlled release of drugs, and not least, as a cosmetic agent. Besides, in view of its biological functions as above mentioned, HA is reported to facilitate, by injection, the nerves regeneration, and when it is placed on wounds it facilitates tissue regeneration. Besides, its characteristics of fast cutaneous permeability and epidermic retention can extend the half-life of drugs administered with it, for example in pharmaceutical devices applied as transdermal administration.

It is evident from the foregoing that biomaterials based on hyaluronic acid, due to its biocompatibility property, are highly suitable as support materials for tissue engineering, useful to facilitate cellular growth processes both *in vivo* and *in vitro,* as well as barriers in the prevention of post-surgical adhesions. Nevertheless, the use of HA alone shows a disadvantage in that it results in scaffolds not very elastic and brittle. Besides, said scaffolds are provided with surfaces too hydrophilic to favour adhesion and cellular differentiation. Especially for this reason it has been several times proposed to modify HA by mixing or crosslinking it with biocompatible polymers as collagen or gelatine, or with synthetic polymers such as polylisine, or chemically modifying HA with hydrophobic groups.

The chemical modification of the polysaccharide molecule of HA by introducing pendent functional groups has as a further target, i.e. to obtain prolonged release pharmaceutical systems (drug delivery systems) where the drug can be carried to the action site while being chemically linked to the polysaccharide carrier chain, and can be released from it in a manner and with times capable to increase its bioavailability.

Another critical drawback of hyaluronic acid used as here considered is its low residence time *in vivo*, due to its fast chemical and enzymatic degradation. In fact, it is degraded by hyaluronidases (HAase), ubiquitous enzymes distributed in human cells and serum, as well as it undergoes a chemical hydrolysis even in the absence of enzymatic activity. For this reason, if on one hand for some applications here reported, the fact that this material could be reabsorbed after performing its function is a required and favourable characteristic, on the other hand it is important that the degradation is not so fast to excessively reduce the product half-life or permanence in the action site.

Therefore, there is a clear interest in developing hyaluronic acid based materials, capable to exploit the advantageous characteristics of this biocompatible material, specifically for the biomedical and pharmaceutical applications reported above, but that, at the same time, have better mechanic and elastic properties and, above all, a better *in vitro* and *in vivo* resistance to chemical and enzymatic hydrolysis, thus performing a prolonged action in the place of application. In addition, as the hydrogels until now considered, the studied materials must have the capability to entrap water and swell in contact with an aqueous medium.

In order to satisfy this demand it has been considered, according to this invention, the opportunity to chemically modify HA by reacting it with a suitable crosslinking agent having a polyaminoacid structure, that is substantially linear and with a protein-like structure, whose biocompatible characteristics have been already ascertatined. In particular, the crosslinking agent proposed according to this invention has a polyhydrazide structure since it shows, for each repeating unit, a pendent hydrazido group (-CO-NH-NH₂), potentially available to covalently link the carboxy group of the repeating disaccharide unit of hyaluronic acid.

The chemical modification of hyaluronic acid by functionalization with bis-hydrazido groups has already been described, for example in the US patents US 5616568 and US 5652347, both to Pouyani et al. (assignee The Research Foundation of State University of New York) and in the corresponding scientific article (T. Pouyani, G. D. Prestwich, Functionalized Derivatives of Hyaluronic Acid Oligosaccharides: Drug Carriers and Novel Biomaterials, Bioconjugate Chem., 1994, 5, 339-347) as well as in Prestwich G D et al: Journal of Controlled Release,vol. 53, no. 1-3, (1998), pages 93-103, and US 5 874 417 A , disclosing HA hydrazide and polyhydrazide derivatives. However, in this case it is not a crosslinking, rather a functionalization of HA, wherein the polysaccharide carboxylic groups reacted with bifunctional groups of general formula H₂N-NH-CO-A-CO-NH-NH₂, where A represents a generic spacer group, to produce a functionalized hyaluronic acid with hydrazido pendent groups: HA-CO-NH-NH-CO-A-CO-NH-NH₂.

The mentioned documents also describe, as concerns the reaction, the known use of a carbodiimide (having general structure R¹-N=C=N-R²) as an agent activating the reaction. For the possible subsequent crosslinking of functionalized HA, in such a manner that the resulting product could form hydrogels, the documents suggest further reactions with a wide range of known crosslinking agents.

In the frame of the same research line, Vercruysse et al. have also proposed (Vercruysse et al., "Synthesis and in Vitro Degradation of New Polyvalent Hydrazide Cross-Linked Hydrogels of Hyaluronic Acid, Bioconjugate Chem., 1997, 8, 686-694) to modify HA by using agents that, having more than two terminal hydrazido groups, could result in a real crosslinking of the starting polysaccharide, to produce materials similar to hydrogels. Despite the work title refers to "polyvalent hydrazides", the reagents considered in the study are synthetic compounds containing from two to six hydrazido functions, and they are not polymeric chains. Apart from the absence of any consideration on the biocompatibility of the bis-, tri-, tetra-, penta- or hexahydrazides employed in the study, the document describes the production of materials having characteristics and structures different from those considered in this invention, firstly because it does not obtain the HA crosslinking by chemical bond with another linear polymer having a different nature, but by using multifunctional reagents with relatively small molecular size.

In view of the foregoing, the present invention proposes to employ as crosslinking agent for HA a polydrazide polymer having a polypeptide backbone, where each repeating unit contains one hydrazido pendent group. In particular, the preferred polymer of the type described is alpha,beta-polyaspartylhydrazide (PAHy), a water-soluble and biocompatible polymeric material, already synthesized and studied by the research group proposing this invention (G. Giammona, B. Carlisi, G. Cavallaro, G. Pitarresi, S. Spampinato, A new water-soluble synthetic polymer, α,β-polyasparthydrazide, J. Control. Rel., 1994, 29, 63-72). This material has been obtained, as reported in the mentioned literature, by aminolysis of a high molecular weight polysuccinimide with hydrazine. In particular, the polysuccinimide (PSI) has been obtained by polycondensation of D,L-aspartic acid, and it has been reacted subsequently with hydrazine (₂HN-NH₂), to obtain a polymer represented by the following chemical formula:

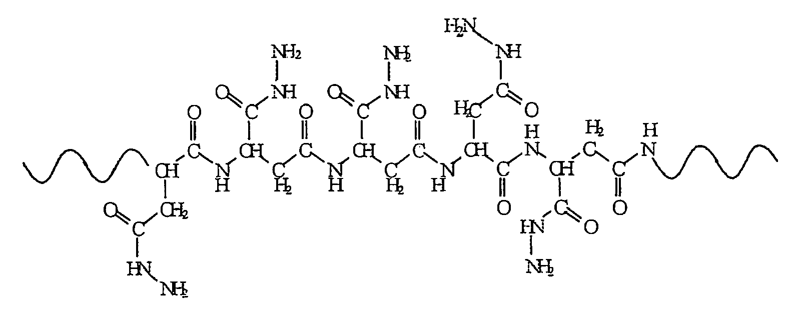

As it is evident from the previous formula, due to the cyclic structure of the starting polysuccinimide, the coupling of hydrazine can occur in such a way as to leave a methylene group either in the polymeric backbone or in the pendent functional group. Therefore, the repeating unit (the above formula shows five repeating units) can have a structure slightly different in the first or in the second case, but its molecular weight is the same.

In the mentioned literature, the synthesis and characterization of PAHy are reported as well as the proposal to use this protein-like polymer as a plasma substitute. For this aim, toxicity studies, immunogenic ability and platelet aggregation tests have been reported and they have demonstrated the total biocompatibility of this polyhydrazido polymer.

Therefore, the present invention specifically provides a composition comprising hyaluronic acid chemically crosslinked with a polyhydrazide polymer, wherein one or more carboxy groups of the disaccharide units of hyaluronic acid are chemically linked respectively to one or more hydrazido groups of the polyhydrazide polymer. Preferably, as pointed out before, said polyhydrazide polymer is alpha,beta-polyaspartylhydrazide (PAHy), that has been exhaustively investigated as concerns its water-solubility, biocompatibility and non-immunogenic properties. However, other polymers with a polyaminoacid structure having an essentially linear chain, with pendent hydrazido groups along the chain, could be employed in the same manner to crosslink hyaluronic acid so as to give compositions and materials with suitable properties of processability, mechanical resistance and resistance to degradation.

In particular, in the composition according to this invention, hyaluronic acid has a molecular weight from 50,000 to 1,500,000 dalton, whereas when the polyhydrazide polymer is PAHy, this has a molecular weight from 2,000 to 40,000 dalton.

According to some preferred embodiments thereof, this invention provides a hydrogel composed of hyaluronic acid chemically crosslinked with a polyhydrazide polymer, as previously defined. Also in this case the polyhydrazido polymer is alpha,beta-polyaspartylhydrazide and, preferably, the hyaluronic acid employed for the hydrogel production has a molecular weight from 50,000 to 1,500,000 dalton, and the alpha,beta-polyaspartylhydrazide has a molecular weight from 2,000 to 40,000 dalton, the most preferred range being from 10,000 to 30,000 dalton.

Preferably, in the production of the crosslinked polymeric material the ratio between moles of repeating unit of alpha, beta-polyaspartylhydrazide and moles of repeating unit of hyaluronic acid is from 0.01 to 5, the most preferred range being from 0,5 to 3.

In view of the chemical structure of both polymers composing the hydrogel according to this invention, it is possible to hypothesise for the product resulting from the crosslinking reaction the following structure:

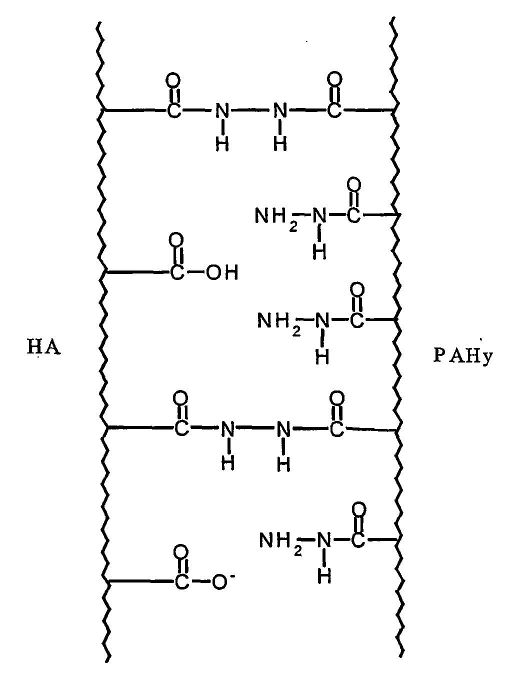

According to some preferred embodiments of the invention, the proposed hydrogels can be obtained by reacting hyaluronic acid with the polyhydrazide polymer in the presence of a carbodiimide (having the general structure R¹-N=C=N-R²) as an activating agent. The preferred activating product is N-ethyl-N'-(3-dimethylaminopropyl)-carbodiimide (EDC), but other similaragents could be employed in its place, such as, for instance, N,N'-dicycloexylcarbodiimide, cyclohexyl-β-(N-methylmorpholine)ethyl carbodiimide p-toluensulphonate (CMC) or N-allyl-N'(β-hydroxyethyl)carbodiimide. Preferably, when EDC is employed the ratio between moles of N-ethyl-N'-(3-dimethylaminopropyl)-carbodiimide and moles of repeating unit of hyaluronic acid is from 0.01 to 10.

To activate the crosslinking reaction, it can be advantageous to add to the reaction medium another activating agent, such as a N-hydroxysuccinimide (NHS), specifically N-hydroxysulfosuccinimide (NHSS). In this case the NHSS is preferably present in the same molar amount as the said N-etil-N'-3-(3-dimethtylaminopropyl)-carbodiimide.

According to another specific aspect thereof, the invention provides a manufacturing process for producing a hydrogel composed of hyaluronic acid chemically crosslinked with a polyhydrazide polymer, wherein the hyaluronic acid, obtained from animal or vegetable sources or by biotechnological processes and having the molecular weight mentioned above, and the polyhydrazide polymer, preferably PAHy, with the molecular weight above reported, are dissolved in double-distilled water in a prefixed molar ratio and a fixed amount of carbodiimide, preferably EDC, is added to them. The reaction mixture is kept from 0°C to 60°C for a time ranging from 1 hour to 5 days, and subsequently the product is recovered as a hydrogel. In this case, during the reaction the pH is maintained in the range between 3 and 8, in particular by using a 0.1 N HCl solution or a solution of bis (2-hydroxyethyl) aminotris (hydroxymethyl)metane hydrochloride with a concentration ranging from 1 to 500 mM.

According to a further aspect thereof, the process according to this invention can include the addition, besides the said carbodiimide, of a prefixed amount of a N-hydroxysuccinimide, preferably NHSS, as a further agent activating the reaction. In this case, during the reaction the pH is maintained in the range between 4 and 10.

After the reaction time, each product is purified by several washings with double-distilled water and then dried by lyophilization (to obtain nanoparticles, microparticles or sponges) or dried for some days at 25°C at a pressure of 1 atm, inside suitable moulds, to obtain films, membranes or rods. As it will more evident below with reference to the reported experimental data, the systems prepared have a wide applicative versatility in the biomedical and pharmaceutical field, and are suitable, as an example, to heal wounds, to prevent post- surgical adhesion, to lubricate joints, to allow the *in vitro* and *in vivo* cell growth, to realize drug delivery systems.

It must be also considered that both the HA and the polyhydrazide polymer solutions could be singly sterilized. After their mixing, the gelation time, that is always greater than 10 minutes, could allow to apply the gel forming solution *in situ* so as to obtain, after a few minutes, the formation of a gel directly on the tissue. In this field there already exist on the market products proposed for the same application, for instance products consisting of a double syringe containing suitable reagents based on PEG (polyethyleneglycol) derivatives, able to crosslink *in situ* after their mixing. The solutions are sprayed on tissues to avoid the post-surgical adhesion.

Thus, the present invention further specifically provides a kit for the *in situ* production of a hydrogel composed of hyaluronic acid chemically crosslinked with a polyhydrazide polymer, preferably alpha,beta-polyaspartylhydrazide, comprising a container with a first hyaluronic acid-based component and a container with a second polyhydrazide polymer-based component, being said two components able to form the hydrogel after their mutual contact, directly on the application site.

Each product obtained according to this invention has been characterized by spectophotometric techniques and swelling measurements in double- distilled water and in media that simulate some biological fluids (extracellular fluid, gastric fluid, intestinal fluid, synovial fluid, aqueous humour or vitreous humour) in a temperature range from 20°C to 40°C. The swelling values, as reported below, have shown a high affinity of the hydrogels prepared according to this invention towards an aqueous medium. The extent of this affinity resulted to be dependent on the crosslinking degree, and on the composition and pH of the swelling medium (investigated pH range from 1 to 9).

Each product of this invention has been also subjected to chemical hydrolysis studies at 37°C, by using media with various saline composition and pH values mimicking some biological fluids (range of investigated pH from 1 to 8) with incubation times from 1 to 30 days. The obtained results, partially reported below, have demonstrated that the proposed products are resistant to chemical hydrolysis as a function of the hydrolysis medium (composition and pH), of the incubation time and the crosslinking degree of the hydrogel.

Finally, the products according to this invention have been subjected to enzymatic hydrolysis studies by using aqueous solutions containing HAase at various concentrations (ranging from 1 to 1000 U/ml), at 37°C and for incubation times ranging from 30 minutes to 30 days. In this case , as reported below, the obtained results have demonstrated that the products of this invention are also resistant to hydrolysis by hyaluronidase, as a function of enzyme concentration, incubation time and crosslinking degree of the hydrogel.

The specific features of the invention, as well as its advantages and the corresponding operating conditions, will be more evident in the detailed description reported below, by way of example only, together with the results of the experiments performed on the invention and the data for comparison with the prior art. Some experimental results are also reported in the attached figures wherein:
Figure 1 shows the swelling behaviour, in aqueous solution with citrate buffer pH 6.3, of a series of sponges based on HA-PAHy hydrogels according to the invention;
Figure 2 shows the swelling behaviour, in aqueous solution with Dulbecco phosphate buffer solution (DPBS) pH 7.4, of a series of sponges similar to those of Figure 1;
Figure 3 shows the results of degradation studies, in the absence of HAase, of a series of sponges based on HA-PAHy hydrogels according to the invention;
Figure 4 shows the results of degradation studies by enzymatic hydrolysis, of a similar series of sponges, wherein the concentration of the employed HAase enzyme is 75U/ml; and
Figure 5 shows the results of degradation studies by enzymatic hydrolysis of a similar series of sponges, wherein the concentration of the employed HAase enzyme is 150 U/ml.

### EXAMPLE 1

An aqueous solution containing hyaluronic acid (0.5 % w/v) has been added to an amount of alpha,beta-polyaspartylhydrazide (PAHy) such as to have a ratio between the moles of repeating unit of PAHy and the moles of repeating unit of hyaluronic acid (ratio indicated as "X") equal to 2.

To activate the reaction between hyaluronic acid and PAHy, N-ethyl-N'-(3-dimethylaminopropyl)-carbodiimide (EDC) alone was employed, in an amount such as to have a ratio between moles of EDC and moles of repeating unit of hyaluronic acid (ratio indicated as "Y") equal to 1.8.

The reaction mixture was kept at 37°C for 4 hours. During the reaction, the pH value was maintained at a constant value of 4.75 by using a solution of bis (2-hydroxyethyl)aminotris(hydroxymethyl)metane hydrochloride with a concentration of 300mM.

After the reaction time, the obtained product was purified by several washings in double-distilled water and then dried by lyophilization to obtain microparticles.

The obtained product has been weighed (yield 94 % ± 1.9) and characterized by spectrophotometric techniques.

By using the same procedure it is also possible to obtain nanoparticles, fims, rods, sponges and gels.

### EXAMPLE 2

The same procedure reported in the Example 1 was repeated with the only difference that the crosslinking reaction was carried out in the presence of EDC as a activant and in the presence of the same molar amount of N-hydroxysulfosuccinimide (NHSS).

In this case the pH value was maintained at 7.5.

By performing the reaction as in Example 1, similar yields were obtained for the purified product, that was characterized by spectrophotometric techniques.

### Swelling studies

The product obtained in various sizes and shapes such as nanoparticles, microparticles, film, rods, sponges and gels, has a high swelling degree in double-distilled water. As reported above, this property confers on the hydrogels of this invention a potential biocompatibility that, in addition, is confirmed by the biocompatibility of both starting polymers, thus giving a wide possibility for the use of these products in the biomedical and pharmaceutical field.

By using a series of HA-PAHy sponges obtained according to the schemes of the previous examples, by varying the molar ratio X and Y defined above, tests of swelling in DPBS buffer pH 7.4 and citrate buffer pH 6.3 were performed. By using suitable procedures, the swelling behaviour was expressed by the ratio (Q) between the equilibrium weight of swollen sponge and the weight of the same sponge dry.

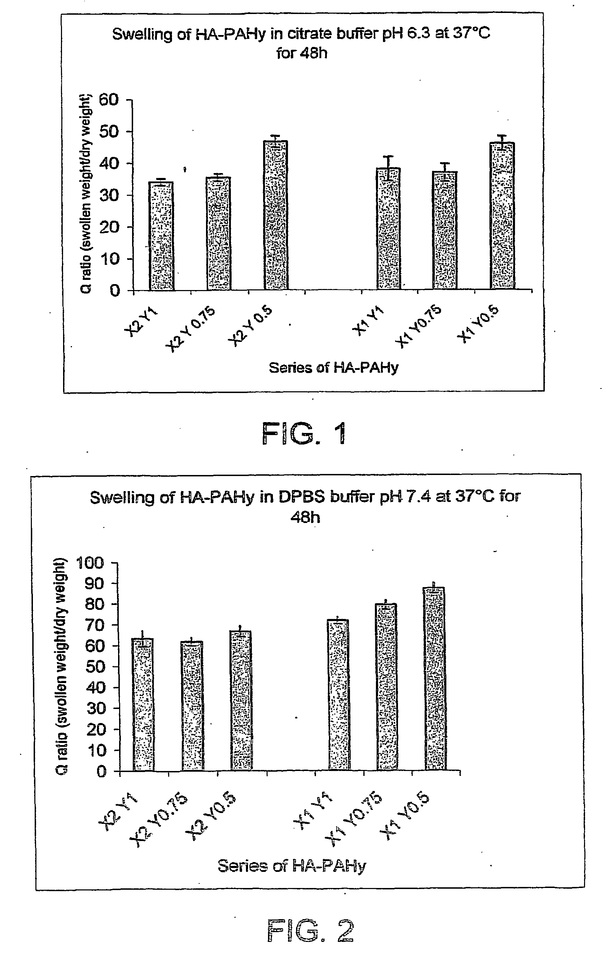

Some results of these experiments are shown in Figures 1 and 2, performed by using two different buffer solutions as aqueous media. It is evident, by examining these figures, that the swelling values in phosphate buffer are twice the corresponding Q values in citrate buffer. Moreover, in both media it is observed that, by increasing the amount of PAHy in the sponges (X ratio between 1 and 2), a slight increase in the swelling ability occurs, this being more evident for smaller Y values.

### Chemical and enzymatic degradation studies

The product obtained according to the schemes of the previous Examples 1 and 2 was subjected to chemical hydrolysis studies at 37°C for 10 days in phosphate buffer solution pH 7.4 (which simulates the extracellular fluid) and pH 5.5 (which simulates the skin pH). After 10 days, the product was recovered, purified by washing with double-distilled water, lyophilized and weighed to determine the percentage of degradation, that resulted to be less than 4%.

For another set of experiments, the series of hydrogels above reported, obtained with various molar ratios X and Y, have been extensively washed and lyophilized, then kept in citrate buffer pH 6.3, in the presence or in the absence of hyaluronidase. The latter was used in two different tests at a concentration of 75 and 150 U/ml, respectively. After incubation at 37°C under stirring for fixed times, the extent of hydrogel degradation was evaluated by using suitable analytical procedures.

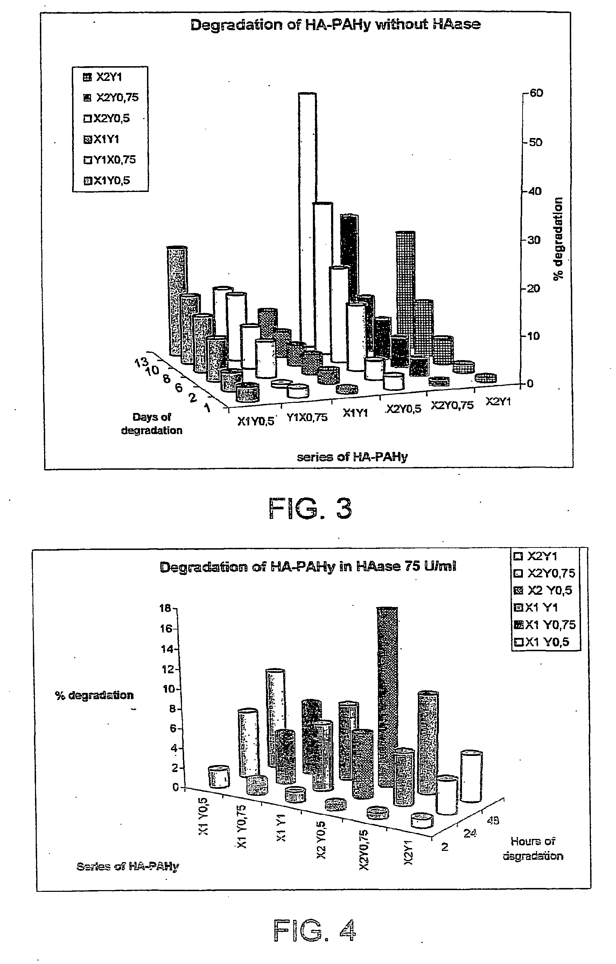

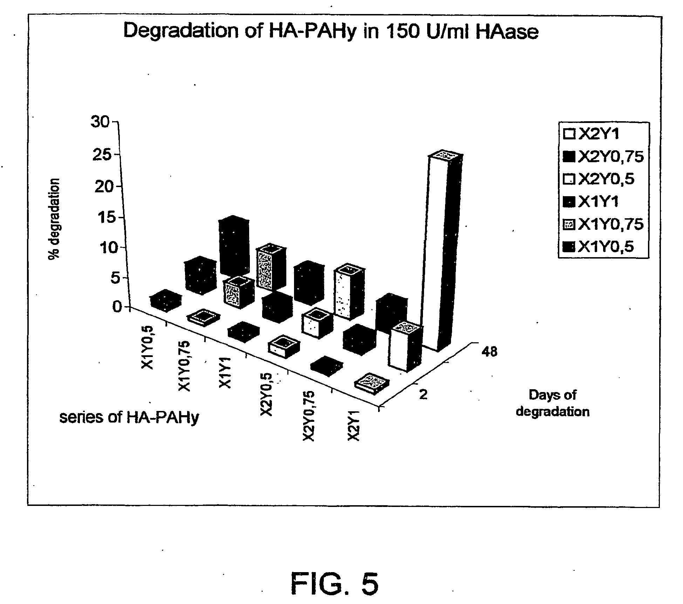

The results of some tests of chemical degradation (in the absence of HAase) and enzymatic degradation with two different concentrations of responsible enzymes, are shown in Figures 3, 4 and 5 respectively. In this case, the tests are also assembled in series as a function of the molar ratios X and Y employed in the hydrogel preparation. By examining these figures, it is evident that for each medium chosen for this experiment, a progressive degradation of the sponges occurs. For each series, with an equal value of X, the degradation decreases by increasing the value of Y. This means, as expected, that the efficiency of crosslinking increases by increasing the amounts of EDC and NHSS.

In addition, with an equal value of Y, it is observed an evident decrease in the percentage of degradation by increasing the amount of crosslinking agent (PAHy), i.e. the value of X, thus demonstrating that the rate of degradation increases by decreasing the crosslinking degree. Finally, it can be observed that the sponge showing the best resistance to degradation is the one obtained with X=1 and Y=1; in fact, after 2 weeks, it shows only a degradation of 10% in the absence of HAase.

Taking into consideration the above results, it is evident that the hydrogels according to this invention possess the advantage to undergo a hydrolytic or enzymatic degradation dependent on time and that can be fixed in advance, as a function of the desired application, by changing the conditions of preparation of the product of this invention. The prepared materials, besides having an excellent compactness and elasticity, are resistant to chemical and enzymatic hydrolysis for several days, but they are totally degradable and reabsorbable after long periods of time.

These advantages are obtained, according to this invention, without penalizing the costs and the ease of production. The latter, on the contrary, is very simple, inexpensive and easily reproducible with high yields. Finally, it must be evidenced that the biomaterials proposed according to this invention represent an excellent combination between the advantages due to biocompatibility of hyaluronic acid and the peculiar properties of a synthetic (artificial) polymer, such as chemical versatility, easy processability and low-cost production.

## Claims

1. A composition comprising hyaluronic acid chemically crosslinked with a polyhydrazide polymer, wherein one or more carboxy groups of the disaccharide units of hyaluronic acid are chemically linked, respectively, to one or more hydrazido groups of the polyhydrazide polymer.

2. A composition according to claim 1, wherein said polyhydrazide polymer is alpha,beta-polyaspartylhydrazide.

3. A composition according to claims 1 or 2, wherein the hyaluronic acid has a molecular weight from 50,000 to 1,500,000 dalton.

4. A composition according to claims 2 or 3, wherein the alpha,beta-polyaspartylhydrazide has a molecular weight from 2,000 to 40,000 dalton.

5. A hydrogel composed of hyaluronic acid chemically crosslinked with a polyhydrazide polymer, wherein one or more carboxy groups of the disaccharide units of hyaluronic acid are chemically linked, respectively, to one or more hydrazido groups of the polyhydrazide polymer.

6. A hydrogel claimed in claim 5, wherein said polyhydrazido polymer is alpha,beta-polyaspartylhydrazide.

7. A hydrogel according to claim 6, wherein the hyaluronic acid has a molecular weight from 50,000 to 1,500,000 dalton and the alpha,beta-polyaspartylhydrazide has a molecular weight from 2,000 to 40,000 dalton.

8. A hydrogel according to claims 6 or 7, wherein the ratio between moles of repeating unit of alpha,beta-polyaspartylhydrazide and moles of repeating unit of hyaluronic acid is from 0.01 to 5.

9. A hydrogel according to any one of claims 5-8, obtainable by reacting hyaluronic acid and a polyhydrazide polymer in the presence of a carbodiimide as an activating agent.

10. A hydrogel according to claim 9, wherein said carbodiimide is N-ethyl-N'-(3-dimethylaminopropyl)-carbodiimide.

11. A hydrogel according to claim 10 wherein the ratio between moles of N-ethyl-N'-(3-dimethylaminopropyl)-carbodiimide and moles of repeating unit of hyaluronic acid is from 0.01 to 10.

12. A hydrogel according to any one of claims 9-11, further obtainable in the presence of a N-hydroxysuccinimide as a further activating agent.

13. A hydrogel according to claim 12 wherein said N-hydroxysuccinimide is N-hydroxysulfosuccinimide.

14. A hydrogel according to claim 13 wherein said N-hydroxysulfosuccinimide is present in the same molar amount as the said N-ethyl-N'-(3-dimethylaminopropyl)-carbodiimide.

15. A manufacturing process for producing a hydrogel as defined in claims 5 or 6, wherein said hyaluronic acid and said polyhydrazide polymer are dissolved in double-distilled water in a prefixed molar ratio and a fixed amount of carbodiimide is added thereto, the resulting reaction mixture is kept at a temperature from 0°C to 60°C for a period of time from 1 hour to 5 days, and subsequently the product is recovered in hydrogel form.

16. A process according to claim 15 wherein said polyhydrazide polymer is alpha,beta-polyaspartylhydrazide and said carbodiimide is N-ethyl-N'-(3-dimethylaminopropyl)-carbodiimide.

17. A process according to claims 15 or 16 wherein during the reaction the pH is maintained in the range from 3 to 8.

18. A process according to claims 15 or 16, wherein in addition to said carbodiimide, a prefixed in advance amount of a N-hydroxysuccinimide is added.

19. A process according to claim 18 wherein said N-hydroxysuccinimide is N-hydroxysulfosuccinimide.

20. A process according to claims 18 or 19 wherein during the reaction the pH is maintained in a range from 4 to 10.

21. Compositions and hydrogels according to any one of the previous claims, in the form of nanoparticles, microparticles, film, membranes, rods, sponges or gels.

22. A kit for the *in situ* preparation of a hydrogel composed of hyaluronic acid chemically crosslinked with a polyhydrazide polymer, comprising a container with a first hyaluronic acid-based component and a container with a second polyhydrazido polymer-based component, the said two components being able to form the hydrogel after their mutual contact, directly on the application site.

23. A kit according to claim 22, wherein said polyhydrazide polymer is alpha,beta-polyaspartylhydrazide.

## Patentansprüche

1. Zusammensetzung, welche Hyaluronsäure umfasst, die chemisch mit einem Polyhydrazidpolymer vernetzt ist, wobei eine Carboxygruppe oder mehrere Carboxygruppen der Disaccharideinheiten von Hyaluronsäure jeweils mit einer Hydrazidogruppe oder mehreren Hydrazidogruppen des Polyhydrazidpolymers chemisch verbunden ist/sind.

2. Zusammensetzung nach Anspruch 1, wobei es sich bei dem genannten Polyhydrazidpolymer um alpha,beta-Polyaspartylhydrazid handelt.

3. Zusammensetzung nach Anspruch 1 oder 2, wobei die Hyaluronsäure ein Molekulargewicht von 50.000 bis 1.500.000 Dalton aufweist.

4. Zusammensetzung nach Anspruch 2 oder 3, wobei das alpha,beta-Polyaspartylhydrazid ein Molekulargewicht von 2.000 bis 40.000 Dalton aufweist.

5. Hydrogel, welches aufgebaut ist aus Hyaluronsäure, die chemisch mit einem Polyhydrazidpolymer vernetzt ist, wobei eine Carboxygruppe oder mehrere Carboxygruppen der Disaccharideinheiten von Hyaluronsäure jeweils mit einer Hydrazidogruppe oder mehreren Hydrazidogruppen des Polyhydrazidpolymers chemisch verbunden ist/sind.

6. Hydrogel, wie nach Anspruch 5 beansprucht, wobei es sich bei dem genannten Polyhydrazidpolymer um alpha,beta-Polyaspartylhydrazid handelt.

7. Hydrogel nach Anspruch 6, wobei die Hyaluronsäure ein Molekulargewicht von 50.000 bis 1.500.000 Dalton und das alpha,beta-Polyaspartylhydrazid ein Molekulargewicht von 2.000 bis 40.000 Dalton aufweist.

8. Hydrogel nach Anspruch 6 oder 7, wobei das Molverhältnis der sich wiederholenden Einheit von alpha,beta-Polyaspartylhydrazid und der sich wiederholenden Einheit von Hyaluronsäure 0,01 bis 5 beträgt.

9. Hydrogel nach einem der Ansprüche 5 - 8, welches durch die Reaktion von Hyaluronsäure und einem Polyhydrazidpolymer in Anwesenheit eines Carbodiimids als Aktivator erhalten wird.

10. Hydrogel nach Anspruch 9, wobei es sich bei dem genannten Carbodiimid um N-Ethyl-N'-(3-dimethylaminopropyl)carbodiimid handelt.

11. Hydrogel nach Anspruch 10, wobei das Molverhältnis von N-Ethyl-N'-(3-dimethylaminopropyl)carbodiimid und der sich wiederholenden Einheit von Hyaluronsäure 0,01 bis 10 beträgt.

12. Hydrogel nach einem der Ansprüche 9 - 11, welches ferner in Anwesenheit von einem N-Hydroxysuccinimid als einem weiteren Aktivator erhalten wird.

13. Hydrogel nach Anspruch 12, wobei es sich bei dem genannten N-Hydroxysuccinimid um N-Hydroxysulfosuccinimid handelt.

14. Hydrogel nach Anspruch 13, wobei das genannte N-Hydroxysulfosuccinimid in der gleichen molaren Menge wie das genannte N-Ethyl-N'-(3-dimethylaminopropyl)carbodiimid vorliegt.

15. Herstellungsverfahren für die Produktion eines Hydrogels, wie in Anspruch 5 oder 6 spezifiziert, wobei die genannte Hyaluronsäure und das genannte Polyhydrazidpolymer in bidestilliertem Wasser in einem zuvor festgelegten molaren Verhältnis aufgelöst werden und eine bestimmte Menge von Carbodiimid hinzugegeben wird, die entstehende Reaktionsmischung bei einer Temperatur von 0 °C bis 60 °C über einen Zeitraum von 1 Stunde bis zu 5 Tagen aufbewahrt wird, und das Produkt anschließend in Hydrogelform gewonnen wird.

16. Verfahren nach Anspruch 15, wobei es sich bei dem genannten Polyhydrazidpolymer um alpha,beta-Polyaspartylhydrazid und bei dem genannten Carbodiimid um N-Ethyl-N'-(3-dimethylaminopropyl)carbodiimid handelt.

17. Verfahren nach Anspruch 15 oder 16, wobei während der Reaktion der pH-Wert in dem Bereich von 3 bis 8 gehalten wird.

18. Verfahren nach Anspruch 15 oder 16, wobei zusätzlich zu genanntem Carbodiimid im Voraus eine zuvor festgelegte Menge von einem N-Hydroxysuccinimid zugegeben wird.

19. Verfahren nach Anspruch 18, wobei es sich bei dem genannten N-Hydroxysuccinimid um N-Hydroxysulfosuccinimid handelt.

20. Verfahren nach Anspruch 18 oder 19, wobei während der Reaktion der pH-Wert in einem Bereich von 4 bis 10 gehalten wird.

21. Zusammensetzungen und Hydrogele nach einem der vorherigen Ansprüche in der Form von Nanopartikeln, Mikropartikeln, Film, Membranen, Stäbchen, Schwämmen oder Gelen.

22. Kit für die In-Situ-Herstellung eines Hydrogels, welches aus Hyaluronsäure, die chemisch mit einem Polyhydrazidpolymer vernetzt ist, aufgebaut ist, umfassend einen Behälter mit einer ersten auf Hyaluronsäure basierenden Komponente und einen Behälter mit einer zweiten auf einem Polyhydrazidpolymer basierenden Komponente, wobei die beiden genannten Komponenten in der Lage sind, nachdem sie miteinander in Kontakt getreten sind, das Hydrogel direkt auf der Applikationsstelle zu bilden.

23. Kit nach Anspruch 22, wobei es sich bei dem genannten Polyhydrazidpolymer um alpha,beta-Polyaspartylhydrazid handelt.

## Revendications

1. Composition comprenant de l'acide hyaluronique réticulé chimiquement avec un polymère polyhydrazide, dans laquelle un ou plusieurs groupes carboxy des unités disaccharides d'acide hyaluronique sont liés chimiquement, respectivement, à un ou plusieurs groupes hydrazido du polymère polyhydrazide.

2. Composition selon la revendication 1, dans laquelle ledit polymère polyhydrazide est l'alpha, bêta-polyaspartylhydrazide.

3. Composition selon les revendications 1 ou 2, dans laquelle l'acide hyaluronique a un poids moléculaire de 50 000 dalton à 1 500 000 dalton.

4. Composition selon les revendications 2 ou 3, dans laquelle l'alpha, bêta-polyaspartylhydrazide a un poids moléculaire de 2 000 dalton à 40 000 dalton.

5. Hydrogel composé d'acide hyaluronique réticulé chimiquement avec un polymère polyhydrazide, dans lequel un ou plusieurs groupes carboxy des unités disaccharides d'acide hyaluronique sont liés chimiquement, respectivement, à un ou plusieurs groupes hydrazido du polymère polyhydrazide.

6. Hydrogel selon la revendication 5, dans lequel ledit polymère polyhydrazide est l'alpha, bêta-polyaspartylhydrazide.

7. Hydrogel selon la revendication 6, dans lequel l'acide hyaluronique a un poids moléculaire de 50 000 dalton à 1 500 000 dalton et l'alpha, bêta-polyaspartylhydrazide a un poids moléculaire de 2 000 dalton à 40 000 dalton.

8. Hydrogel selon les revendications 6 ou 7, dans lequel le rapport entre les moles d'unité de répétition d'alpha, bêta-polyaspartylhydrazide et les moles d'unité de répétition d'acide hyaluronique est de 0,01 à 5.

9. Hydrogel selon l'une quelconque des revendications 5 à 8, qui peut être obtenu en faisant réagir l'acide hyaluronique avec un polymère polyhydrazide en présence d'un carbodiimide en tant qu'un agent d'activation.

10. Hydrogel selon la revendication 9, dans lequel ledit carbodiimide est le N-éthyl-N'-(3-diméthylaminopropyl)-carbodiimide.

11. Hydrogel selon la revendication 10, dans lequel le rapport entre les moles de N-éthyl-N'-(3-diméthylaminopropyl)-carbodiimide et les moles d'unité de répétition d'acide hyaluronique est de 0,01 à 10.

12. Hydrogel selon l'une quelconque des revendications 9 à 11, qui peut en outre être obtenu en présence d'un N-hydroxysuccinimide en tant qu'un agent d'activation supplémentaire.

13. Hydrogel selon la revendication 12, dans lequel ledit N-hydroxysuccinimide est le N-hydroxysulfosuccinimide.

14. Hydrogel selon la revendication 13, dans lequel ledit N-hydroxysulfosuccinimide est présent dans la même quantité molaire que ledit N-éthyl-N'-(3-diméthylaminopropyl)-carbodiimide.

15. Procédé de fabrication pour produire un hydrogel selon les revendications 5 ou 6, dans lequel ledit acide hyaluronique et ledit polymère polyhydrazide sont dissous dans de l'eau à double distillation dans un rapport molaire prédéterminé et une quantité déterminée de carbodiimide y est ajoutée, le mélange réactionnel résultant est maintenu à une température de 0°C à 60°C pendant une période de temps d'1 heure à 5 jours, et ultérieurement le produit est récupéré sous forme d'hydrogel.

16. Procédé selon la revendication 15, dans lequel ledit polymère polyhydrazide est l'alpha, bâta-polyaspartylhydrazide et ledit carbodiimide est le N-éthyl-N'-(3-diméthylaminopropyl)-carbodiimide.

17. Procédé selon les revendications 15 ou 16, dans lequel pendant la réaction le pH est maintenu dans le domaine de 3 à 8.

18. Procédé selon les revendications 15 ou 16, dans lequel en plus dudit carbodiimide, une quantité prédéterminée à l'avance d'un N-hydroxysuccinimide est ajoutée.

19. Procédé selon la revendication 18, dans lequel ledit N-hydroxysuccinimide est le N-hydroxysulfosuccinimide.

20. Procédé selon les revendications 18 ou 19, dans lequel pendant la réaction le pH est maintenu dans le domaine de 4 à 10.

21. Compositions et hydrogels selon l'une quelconque des revendications précédentes, sous la forme de nanoparticules, microparticules, film, membranes, tiges, éponges ou gels.

22. Kit pour la préparation *in situ* d'un hydrogel composé d'acide hyaluronique réticulé chimiquement avec un polymère polyhydrazide, comprenant un conteneur avec un premier composant à base d'acide hyaluronique et un conteneur avec un second composant à base de polymère polyhydrazide, lesdits deux composants étant capables de former l'hydrogel après leur contact mutuel, directement sur le site d'application.

23. Kit selon la revendication 22, dans lequel ledit polymère polyhydrazide est l'alpha, bêta-polyaspartylhydrazide.
